# EUROPEAN PATENT SPECIFICATION

(11) **EP 4 048 680 B1**
(45) Date of publication and mention of the grant of the patent: **31.01.2024**
(21) Application number: 20811090.8
(22) Date of filing: 23.10.2020
(51) Int. Cl.: C07K 7/06, C12N 9/22, A61K 8/64, A61K 8/14, A61K 8/24, A61K 8/34, A61K 8/365, A61K 8/49, A61K 8/60, A61P 17/00, A61Q 17/04, A61Q 19/02, A61Q 19/08, A61K 38/00

(54) **PEPTIDES WITH CELL DAMAGE PROTECTION ACTIVITY AND LIPOSOMIAL FORMULATIONS**
PEPTIDE MIT ZELLSCHÄDIGUNGSSCHUTZAKTIVITÄT UND LIPOSOMENFORMULIERUNGEN
PEPTIDES AYANT UNE ACTIVITÉ DE PROTECTION CONTRE LES DOMMAGES CELLULAIRES ET FORMULATIONS LIPOSOMALES

(30) Priority: 23.10.2019 IT 201900019667
(43) Date of publication of application: 31.08.2022
(73) Proprietor: I.R.A. Istituto Ricerche Applicate S.p.A., 20865 Usmate Velate (IT)
(72) Inventor: CITERNESI, Ugo Raffaello, 23881 Airuno (IT); ANDOLINA, Gloria, 20865 Agrate Brianza (IT); FORMAGGIO, Fernando, 36025 Noventa Vicentina (IT); PEGGION, Cristina, 35010 San Giorgio delle Pertiche (IT); DI LIDDO, Rosa, 45100 Rovigo (IT); PICCIONE, Monica, 70016 Noicattaro (IT); BERETTA, Lorenzo, 20855 Lesmo (IT); CIVIDINI, Andrea, 20161 Milano (IT); DE MARNI, Emanuele Giuseppe, 20862 Arcore (IT); CITERNESI, Lorenzo, 20866 Carnate (IT)
(74) Representative: Metroconsult Srl
(86) International application number: PCT/IB2020/059968
(87) International publication number: WO 2021/079328

(56) References cited:
- EP-A1- 3 061 815
- WO-A1-2009/097568
- WO-A2-2007/113531
- US-A- 5 308 762
- US-A- 5 985 829
- BASILICO G ET AL: "UV-specific DNA repair recombinant fusion enzyme: A new stable pharmacologically active principle suitable for photoprotection", JOURNAL OF DERMATOLOGICAL SCIENCE, ELSEVIER, AMSTERDAM, NL, vol. 39, no. 2, 1 August 2005 (2005-08-01) , pages 81-88, XP027793039, ISSN: 0923-1811 [retrieved on 2005-08-01]
- JUN SEOK YU ET AL: "The Spectroscopic Study on the Role of C-terminal Region of T4 endonuclease V in the Interaction with DNA: NMR and Fluorescence Experiment", YAKHAK HOEJI, vol. 40, no. 2, 1 December 1996 (1996-12-01), pages 193-201, XP055705294,

## Description

### FIELD OF THE INVENTION

The invention relates to peptides and compositions containing them and the use thereof to limit or correct cell damage caused by external agents such as solar irradiation, environmental stress and/or aging of the epidermal cells.

The use of peptides and of the liposomal formulations thereof to prevent and limit skin damage caused by the exposure to ultraviolet rays in particular in subjects affected by xeroderma pigmentosum is also described. The peptides and the formulations thereof also find application in the cosmetic field.

### STATE OF THE ART

The skin is the outermost tissue of the human body and as such it is the tissue that is mostly exposed to the action of external agents such as solar radiations, atmospheric agents and environmental pollution.

The daily action of these external agents contributes in a decisive way not only to accelerate the physiological aging process of the skin but also to favour the development of skin diseases and skin precancerous or cancerous forms. Recent studies have shown that the latter, in most cases, are attributable to a damage of the cellular DNA caused by the exposure to solar radiations, in particular ultraviolet radiations.

Photoexposure has been shown to cause damage to cellular DNA leading to the formation of pyrimidine photoproducts and cyclobutane pyrimidine dimers (CPD).

These photoproducts can also form when the skin is exposed to UV radiations for a short time. For this reason, the different repair mechanisms capable of removing damaged DNA play a decisive role in the proper functioning of the cell.

In the absence or in case of reduction of these mutation control processes, epithelial cells can easily be affected by neoplasms induced by UV radiations.

Significant evidence of the importance of DNA repair mechanisms is obtained from observing patients affected by Xeroderma pigmentosum (XP).

XP is a rare, autosomal and recessive genetic disease characterized by an enzyme defect in the early stages of the *pathway* for the repair of the genetic damage induced by irradiation. The result is extreme sensitivity to the sun, which entails a high risk of sunburn, hypo- and/or hyper-pigmentation phenomena, early actinic keratosis and multiple skin neoplasms. In the first years of life, patients show an abnormal and significantly marked erythemic response to sunlight. By the age of 20, about 90% of people with this condition have developed at least a cancerous form of the skin.

Over forty years ago, Tanaka and colleagues demonstrated that the enzyme endonuclease T4 V, a 16.5 KDa polypeptide isolated from *Escherichia coli* infected with bacteriophage T4, is able to increase cellular repair by excision of nucleotides in human cells and initiate the removal of CPDs. Since then the gene *denV,* which codes for the enzyme, was cloned and sequenced.

Studies have demonstrated that the enzyme alone is capable of replacing the multi-enzyme complex used for nucleotide excision repair, both when it is expressed in eukaryotic cells by transfection of the gene *denV,* and if it is administered intracellularly as a heterologous enzyme isolated from *E. coli* The studies also documented significant results, both *in vivo* and *in vitro,* on the capability of this enzyme to effectively restore cell damage induced by irradiation to UV rays.

These observations have led to the development of topical formulations of the enzyme endonuclease T4 V (T4N5), which are suitable for carrying the enzyme inside the cell. Several studies have demonstrated that the topical formulation T4N5 crosses the stratum corneum and penetrates the deepest layers of the epidermis, accumulating in the keratinocytes and in the Langerhans cells.

In the course of clinical trials, the topical application of the formulation T4N5 has demonstrated to contribute to the elimination of CPDs at the application site and has demonstrated to be effective in preventing the development of actinic keratosis and carcinomas in basal cell in XP patients.

The protein endonuclease T4 V appears as a single compact domain, with two distinct sites for catalytic activity:
(i) a site functions as glycosylase of the pyrimidine dimer, that is, it identifies the damaged part of DNA and cleaves the dimer formed by UV radiations, eliminating the mutated base;
(ii) the second site removes the sugar and the phosphate from the same glycoside.

Mutations inserted for identifying residues indispensable for enzymatic activity have demonstrated that the carboxy-terminus region of the sequence is essential for recognising and binding the CPDs.

At present, there is a need to have active ingredients whose action is to repair a cell damage, particularly induced by exposure to external agents, typically ultraviolet radiations.

One of the objects of the invention therefore consists in providing active ingredients with biological activity for the therapeutic treatment of skin diseases caused by exposure to external agents in particular to ultraviolet radiations.

Another object of the invention is to provide active ingredients with biological activity for the cosmetic treatment of the skin and for delaying the physiological skin aging process.

Another object of the present invention is to provide formulations for topical application in order to carry peptides having cellular repair activity in the skin layers and inside the cells.

Reference D1 EP 3 061 815 A1 (CONSEJO SUPERIOR INVESTIGACION [ES] ET AL.) 31 August 2016 (2016-08-31) is regarded as being the prior art closest to the subject-matter of claim 1, and discloses an 11-mer peptide derived from dyskerin for use in the treatment of conditions caused by DNA damage by UV radiation including xeroderma pigmentosum (see paragraph [0008], SEQ ID NO: 15 and claims 1 to 15). The subject-matter of independent claim 1 therefore differs from this known peptide in that the peptide consists of the amino acid sequence represented by general sequence SEQ ID NO: 1.

### SUMMARY OF THE INVENTION

The present invention originates from having identified selected biologically active peptides that increase the speed of physiological processes/mechanisms of repair of damaged DNA in mammalian cells and/or contribute to the removal of cyclobutane pyrimidine dimers and/or pyrimidine photoproducts at the level of cellular DNA.

The authors have also found that the biologically active peptides of the invention exert their activity even when they are supplied intracellularly.

In a first aspect, the present invention therefore relates to a peptide consisting of the sequence SEQ ID NO:1, or a salt thereof.

The peptide of SEQ ID NO:1 consists of the sequence, from the N-terminus end to the C-terminus end, represented by the general formula (1):

(1) R¹-**Y**-Xaa¹-Xaa²- Xaa³-Xaa⁴- Xaa⁵-Xaa⁶- Xaa⁷-Xaa⁸- Xaa⁹-Xaa¹⁰

wherein:
Xaa ¹:Trp, Phe or Tyr;
   Xaa ²:Trp, Phe or Tyr;
   Xaa³:Lys;
   Xaa ⁴:Tyr, Trp, or Phe;
   Xaa ⁵:Tyr, Trp, or Phe;
   Xaa ⁶:Gly or Ala;
   Xaa ⁷:Lys;
Xaa ⁸:Ala, or Aib (α-aminoisobutyric acid);
   Xaa ⁹:Ile, Leu or Ala;
   Xaa ¹⁰:Tyr;
   **Y,** if present, is Aib
   and wherein
**R¹**, if present, is H, CF (carboxy fluorescein), palmitoyl (CH₃(CH₂)₁₄CO-), oleyl (CH₃(CH₂)₇(CH)₂(CH₂)₇ CO-), stearyl (CH₃(CH₂)₁₆CO-), linoleyl (CH₃(CH₂)₄(CH)₂CH₂(CH)₂(CH₂)₇CO-), or linolenyl (CH₃CH₂(CH)₂CH₂(CH)₂CH₂(CH)₂(CH₂)₇CO-).

The substituents Y and R ¹ are optional.

The peptides according to the present invention have a structure which gives them a considerably higher simplicity of production than the enzyme endonuclease T4 V, making the molecules suitable for applications as active ingredients.

Furthermore, the peptides of the invention exhibit a significant cellular activity but different from the one of the enzyme T4 V. The inclusion of non-natural amino acids, for example like Aib or lipid chains, further increases the stability to enzymatic degradation.

These properties give the peptides of the invention a high repair activity of the skin cells which allows them to be used both for medical and cosmetic applications.

In accordance with an embodiment of the invention, the peptide having the sequence SEQ ID NO:2, the salts thereof having the following amino acid sequence is provided:
H₂N-Aib-Trp-Phe-Lys-Tyr-Tyr-Gly-Lys-Ala-Ile-Tyr

This selected peptide has a high capacity of recognition and binding of CPDs.

In accordance with specific embodiments of the invention, the following preferred peptides (Ptd-1-7) are provided, the salts thereof have the following sequences SEQ ID NO.2-8:

| SEQ ID NO: | | Sequence | Symbol |
|---|---|---|---|
| 3 | | WFKYYGKAIY | Ptd-1 |
| 4 | | CF-WFKYYGKAIY | Ptd-2 |
| 5 | | Pal-WFKYYGKAIY | Ptd-3 |
| 6 | | WFKYYGK-Aib-IY | Ptd-4 |
| 7 | | Pal-WFKYYGK-Aib-IY | Ptd-5 |
| 8 | | CF-WFKYYGK-Aib-IY | Ptd-6 |
| 2 | | Aib-WFKYYGKAIY | Ptd-7 |

In the medical field, peptides find application in the treatment and protection of cells against damage induced by aggressive external agents, in particular by UV radiations and for the treatment of skin diseases resulting from exposure to UV rays. In the cosmetic field, the peptides of the invention are used in the prevention and/or treatment of the marks of skin aging or skin imperfections resulting from aging and/or the action of external agents such as ultraviolet radiations.

The inventors have also found that liposomes are a suitable system for the transdermal and intracellular delivery of the peptides of the invention. In particular, the inclusion of peptides inside phospholipid vesicles such as liposomes, allows the peptides to be carried through the skin layers and inside the cell where they exert their activity.

The peptides carried or formulated with liposomes penetrate the deepest layers of the epidermis accumulating in the keratinocytes and in the Langherans cells.

In accordance with one aspect, the present invention therefore relates to a composition comprising peptides with sequence SEQ ID NO:1, the salts thereof and a carrier for transporting the peptides through the skin layers.

Preferably the composition for the application comprises a peptide with SEQ ID NO:2 and/or SEQ ID NO:3, the salts thereof and a physiologically acceptable carrier, preferably liposomes.

In one embodiment the composition comprises a peptide having the sequence SEQ ID NO:2-8, the salts thereof and liposomes and a physiologically acceptable carrier, preferably liposomes.

Preferably the composition is for topical use.

The inventors have also observed that the inclusion of the peptides of the invention inside the liposomes performs the dual function of transport through the skin layers and through the cell membrane of the target cells, typically Langherans cells and keratinocytes.

The use of liposomes as a system for transdermally carrying the active peptides of the invention entails advantages such as a high capacity to transport hydrophilic and lipophilic molecules, high penetrability in the cell thanks to affinity and compatibility with biological membranes, high safety of use in combination with moistening and regenerating properties on the stratum corneum of the skin.

The composition of the invention can be a pharmaceutical composition or a cosmetic The references to methods of treatment in the subsequent paragraphs of this description are to be interpreted as references to the compounds, pharmaceutical compositions and medicaments of the present invention for use in a method for treatment of the human (or animal) body by therapy.

The use of the composition comprising one or more peptides, each other equal or different, of SEQ ID NO:1-8 described above and the salts thereof, as a medicament is also described.

The peptides and peptide compounds are active in preventing and/or treating diseases and pathological conditions associated with a damage to the DNA of the cells, in particular of the skin tissue, for example induced by exposure to UV rays. A further aspect of the invention relates to a composition comprising one or more peptides, each other equal or different, of SEQ ID NO:1 -8, the derivatives or salts thereof, for use in the prevention and/or treatment of skin tumours.

Another aspect concerns a composition comprising one or more peptides, each other equal or different, of SEQ ID NO:1-8, the salts thereof, for use in preventing and/or treating xeroderma pigmentosum (XP).

The composition comprising one or more peptides, each other equal or different, of SEQ ID NO:1-8, the salts thereof, is also used in the prevention and treatment of sunburn, erythema, hypo- and/or hyper- pigmentation phenomena, early actinic keratosis and skin neoplasms.

According to a further aspect, the present invention relates to the use of a cosmetic composition comprising one or more peptides, each other equal or different, of SEQ ID NO:1-8, the salts thereof, to prevent and/or treat the marks of skin aging or skin imperfections in particular resulting from skin aging and/or the action on the skin of external agents such as ultraviolet radiations.

In accordance with another aspect, the invention relates to the cosmetic use of one or more peptides, each other equal or different, of SEQ ID NO:1-8, the salts thereof, or of a cosmetic composition for topical application that contains them, in the treatment of a skin imperfection.

The cosmetic composition of the invention is mainly applied in the treatment of spots and/or hyperpigmentation and/or skin dyschromia.

The cosmetic composition is also indicated in the prevention and/or treatment of the marks of skin aging resulting from exposure to UV rays such as corrugations and wrinkles in particular of the face.

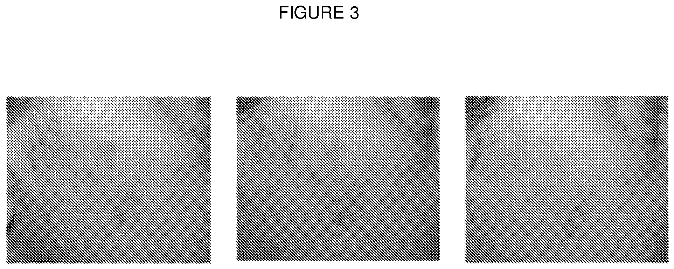

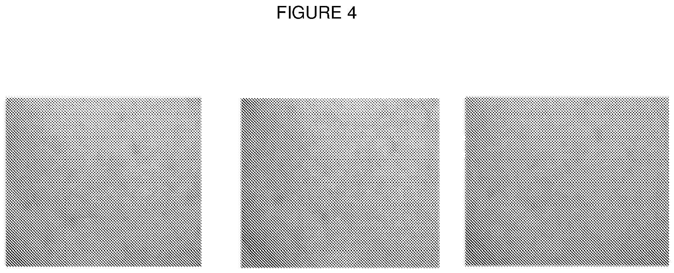

Advantageously, the composition described herein acts by lightening the spots or dyschromia of the skin, as illustrated in the attached figures 3 and 4 and in the following detailed description of the invention.

In accordance with an aspect, the invention provides a cosmetic kit for the treatment of skin marks or skin dyschromia or spots comprising a composition as described herein.

In accordance with an embodiment, the Kit for the cosmetic treatment of the skin comprises
- a cosmetic composition for peeling skin, preferably comprising a α-hydroxy acid preferably glycolic acid, mandelic acid or kojic acid;
- a composition containing one or more peptides, each other equal or different, of SEQ ID NO:1-8;
- a composition preferably in gel form comprising liposomes and S-arbutin and liposomes; and optionally
- a post-peeling buffer composition preferably in solution form;
- a composition for UV-ray protection preferably in cream form.

### DESCRIPTION OF THE DRAWINGS

The invention will now be described in detail and with reference to the following accompanying Figures:

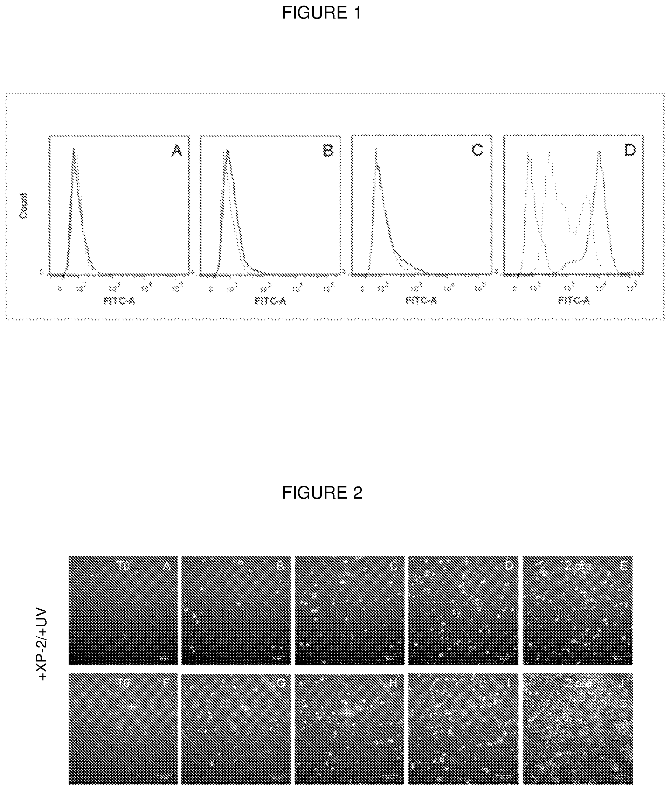

Figure 1 reports the results of the flow cytometric analysis of the fluorescent signal in cells KB-31 treated for 72 hours with liposomal formulations as defined in Example 1. In the images shown, the grey (lighter) profile indicates the signal of the expanding cells **A.** Black profile: cells treated with XP-0 (liposomal formulation not containing peptides as per Example 1); **B.** Black profile: cells treated with XP-1 (liposomal formulation as per Example 1); **C.** Black profile: cells treated with XP-T4V; **D.** Green profiles indicate cells treated with XP-2 (liposomal formulation containing the peptide functionalized with the carboxy fluorescein fluorophore as per Example 1).Light green:XP-2 10-40 µL/mL; Dark green:XP-2 50 µL/mL.
Figure 2 reports Analysis of live cell imaging by confocal microscopy. XPC-F were treated with the formulation XP-2 and irradiated (300-800nm, 360 W/m ² for 7 minutes) with SunTest CPS ⁺ (ATLAS, Urai, Italy).At the end of the irradiation, the samples were observed for 2 hours under controlled conditions (37°C, 5% CO ₂) using a Cell-Observer (Zeiss) system. The cells treated with the formulation in the absence of irradiation (A-E) were used as controls. The fibroblasts treated with formulation XP-2 and irradiated are shown in Figs. F-J.
Figure 3 shows the representative images of the results obtained on skin spots of an individual treated using the comprehensive exfoliating and depigmenting cosmetic kit described in Example 3.
Figure 4 shows the representative images of the results obtained on the complexion of an individual treated using the comprehensive exfoliating and depigmenting cosmetic kit described in Example 3.

### DETAILED DESCRIPTION OF THE INVENTION

In one aspect the invention originates from having found and synthesized peptides which favour the repair of the DNA of the epidermal cells, in particular DNA damaged by exposure to sun radiation.

The authors observed how the peptides described herein favour the repair of the DNA damaged by exposure to sun rays or carcinogenic substances with the nucleotide excision repair (NER) mechanism.

In the context of this acronym T4N5 designates the enzyme endonuclease T4 V.

In accordance with a first aspect, the present invention therefore provides a peptide as defined in the attached claim 1.

Preferred peptides among those falling within the general formula are defined in the attached claim 2.

A further preferred peptide is defined in the attached claim 3.

In the present invention when the following definitions are used:
- "-Aib-" is intended as comprising the non-coded natural amino acid: alpha-aminoisobutyric acid;
All the other amino acids are described with their three letter code for example Gly=glycine, Leu=leucine, Ala=alanine.

All chiral amino acids have a L configuration if not specified.

The peptides can be used alone or in combination and can be prepared in the form of a composition. They can be prepared as an aqueous solution and can be dissolved together with substances adapted to prolong their persistence or adhesion to plant surfaces.

Peptides are also described in which at least one amino acid residue has been removed and preferably only one, and a different residue has been inserted in its place. For a detailed description of protein chemistry and structure see Schulz G.E. et al., 1979 (7) and Creighton T.E., 1984 (8).The substitutions that can be made to the peptide molecule of the present invention are conservative substitutions and are defined herein as exchanges within one of the following groups:
1. Aliphatic, non-polar or slightly polar residues: Ala and Gly;
2. Positively charged polar residues: Lys.

Substantial changes in the functional properties are made with less conservative substitutions, among amino acids belonging to different of the above groups (or two other amino acid groups not shown above), rather than among amino acids of the same group, which differ more significantly in their effect on the maintenance (a) of the structure of the backbone of the peptide in the substitution area (b) of the charge or hydrophobicity of the molecule at the target site, or (c) of the encumbrance of the side chains. Most of the substitutions according to the present invention are those which do not produce radical changes in the characteristics of the peptide molecule.

Even when it is difficult to predict the exact effect of a substitution before producing it, a person skilled in the art will appreciate that the effect can be assessed by routine screening assays, preferably the biological tests described below. The modifications in the properties of the peptide including redox or thermal stability, hydrophobicity, susceptibility to proteolytic degradation, or the tendency to aggregate with carriers or in multimers are tested with methods well known to the person skilled in the art.

"Chemical derivatives" of the general formula (1) SEQ ID NO:1 containing additional chemical subunits which are not normally part of the peptide. The covalent modifications of the peptide are included in the scope of the present invention. Such modifications can be introduced into the molecule by reacting suitable amino acid residues of the peptide with an organic derivatizing agent which is capable of reacting with selected side chains or terminus residues.

Other examples of chemical derivatives of the peptide follow.

The N-terminus residue of Lysine can be derivatized with succinic acid or other carboxylic acid anhydrides. Derivatization with cyclic carboxylic anhydride has the effect of reversing the charge of the lysine residues. Other suitable reagents for derivatization of residues containing α-amino groups comprise imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

The carboxyl, aspartyl or glutamyl side groups can be selectively modified by reaction with carbodiimides (RN = C = N-R') such as 1-cyclohexyl-3-(2-morpholinyl-(4-ethyl) carbodiimide or 1-ethyl-3-(4-azonia-4,4-dimethylpentyl) carbodiimide. Furthermore, aspartyl and glutamyl residues can be converted into asparaginyl and glutaminyl residues by reaction with ammonia.

Other modifications include hydroxylation of proline and lysine, phosphorylation of hydroxyl groups of seryl or threonyl residues, methylation of the amino group of lysine (Creighton, above, pages 79-86), acetylation of the N-terminus amine, and amidation of the C-terminus carboxyl groups.

Peptides are also included in which one or more L-amino acids have been substituted with one or more D-amino acids.

Peptides are also included in which one or more D-amino acids have been substituted with one or more L-amino acids. In addition, modified amino acids or chemical derivatives of amino acids can be provided such that the peptide contains additional chemical components or modified amino acids that are not normally part of a natural protein. Such derivatized functions can improve solubility, absorption, biological half-life and the like. Derivatizations that are capable of mediating such effects are reported, for example, in Remington's Pharmaceutical Sciences 1980 (9).

Peptides with the sequences described are included in which one or more amide functions are N-methylated.

The peptides of the invention can be prepared with a general process based on the solid phase peptide synthesis preferably using amino acids protected by a protecting group Fmoc ( *Fluorenylmethyloxycarbonyl*)*.*

Typically, the peptides can be prepared by solid phase peptide synthesis, with solid support typically functionalised to obtain the carboxylic acid at the C-terminus end, for example by using acid labile Wang type resins or super acid labile resins of the Cl-Trt or Sasrin type.

In the case of use of Cl-Trt resin, the first amino acid, Fmoc-Tyr(tBu)-OH is dissolved in a suitable solvent, typically DMF and bound to the resin preferably in the presence of DIPEA. A capping procedure follows preferably using a DCM:MeOH:DIPEA mixture, for example 80:15:5 for 10 minutes.

The Fmoc groups for the NH-protection can be eliminated by a treatment, typically lasting 7-9 minutes, with piperidine for example at 20% in DMF followed by a second treatment with the same reagent for example for 8-12 minutes.

The coupling of each amino acid is obtained by reacting 3 equivalents of Fmoc-AA-OH with respect to the moles of active sites of the resin. Preferably the activation of the carboxyl function is obtained with 3 excess equivalents of DIC and 3 excess equivalents of Oxyma-Pure. Preferably, the coupling reaction is conducted using carbodiimides as activators in the presence of HOBt/HOAt or uronium salts such as TBTU, HATU, HBTU. Advantageously, the coupling reaction is performed in a DMF solution for example for 1 hr followed by a washing procedure, for example 6 washes with DMF. Deprotection of the amino function of each Fmoc-AA-OH is obtained by means of a double treatment with a 20% piperidine solution in DMF, followed by the removal of excess reagents by filtration and washing procedure. The process is repeated for each new residue in the peptide chain.

It is possible to check the success of each coupling with the Kaiser test. At the end of the synthesis of the entire sequence, the peptide resin can be washed with DMF (×3), DCM (×4) and then dried preferably under vacuum.

According to an embodiment, in order to obtain the detachment from the resin and the elimination of the protecting groups from the peptide chain, the resin is treated, for example for 3 hours, at room temperature with a cleavage cocktail (trifluoroacetic acid/H₂O/triisopropylsilane = 95/2.5/2.5 v/v, 3 ml/100 mg of resin).After the cleavage reaction, the product can be precipitated by adding cold ether, and then allowed to dry under vacuum at 50°C.

The raw product was purified by preparative RP-HPLC, in the presence of 0.05% - 0.1% TFA. The harvested eluate can be freeze-dried. It is possible to obtain the pure product both as a trifluoroacetate salt and as a chloride salt following repeated lyophilization from diluted HCl.

The peptides thus obtained were characterized by RP-HPLC and mass spectrometry (ESI-TOF).

| Nam e | Sequence | SEQ ID NO: | r.t.* | theoretical MW ^{#} | MW^{#} [M+H⁺] | Purity |
|---|---|---|---|---|---|---|
| Ptd-1 | WFKYYGKAIY | 3 | 5.4 | 1338.58 | 1338.79 | 99.9 % |
| Ptd-2 | CF-WFKYYGKAIY | 4 | 13.3/13.9 | 1696.09 | 1697.60 | 97.7 % |
| Ptd-3 | Pal-WFKYYGKAIY | 5 | 26.6 | 1576.96 | 1576.90 | 95.0% |
| Ptd-4 | WFKYYGK-Aib-IY | 6 | 5.8 | 1352.59 | 1352.50 | 97.8 % |
| Ptd-5 | Pal-WFKYYGK-Aib-IY | 7 | 26.8 | 1591.91 | 1590.90 | 99.9 % |
| Ptd-6 | CF-WFKYYGK-Aib-IY | 8 | 13.5/14.1 | 1710.90 | 1710.70 | 83.1 % |
| Ptd-7 | Aib-WFKYYGKAIY | 2 | 7.2 | 1422.70 | 1423.70 | 96.1 % |
| | Pal: palmitoyl; Aib: 2-aminoisobutyric acid; CF: carboxy fluorescein. | | | | | |

| | | | | | | |
|---|---|---|---|---|---|---|
| *r.t.: retention time on column Phenomentex, Kinetex XB-C18 100Å 4.6 × 100 mm, flow 1 mL/min; gradient 20-80% Buffer B in 30 min. Buffer A:0.1% TFA in H₂O Buffer B:0.1% TFA in CH ₃ CN.^{#} MW: molecular weight. | | | | | | |

In accordance with another aspect, the present invention provides a composition as defined in the attached claims 4-6.

The composition of the invention comprises a peptide as described herein and a physiologically acceptable carrier.

In the context of the present invention, the term "carrier" refers to an excipient, carrier, diluent or adjuvant which may or all of them may be present in the composition of the invention. Any carrier and/or excipient can be used, in particular if suitable for topical administration of the composition.

Preferably the carrier is physiologically acceptable, it facilitates the crossing of the skin barrier and allows to reach the deepest layers of the epidermis where the peptides exert their repairing action.

Within the scope of the invention, a preferred carrier are liposomes.

Typically, the liposomes used are based on vesicles comprising one or more lipid bilayers, which enclose an aqueous environment. The liposomal, mimetic membrane of the cell membrane, comprises a mixture of phospholipids, or phospholipids and cholesterol, which give the particle advantages of biocompatibility and high capacity of membranes penetration and physiological barriers.

Liposomes behave like systems encapsulating the peptides, which are thus physically isolated from the surrounding environment and therefore protected from any chemical or enzymatic degradation processes before they reach the target area.

In the composition described herein, the liposomes can play the dual role of carrier of peptides, and of biologically active substances. For example, in the case of erythema or skin dryness, the liposomes can interact with the lipids, the proteins and the carbohydrates of the skin, with a beneficial, moisturising and restorative effect on the stratum corneum.

The liposomes present in the formulation of the composition carry the peptides through the epidermis and the underlying skin layers up to a cellular level, overcoming the cell membrane. The liposomes maintain their moistening and regenerative capacities and contribute to increasing the penetration, the solubility or the stability of other substances, isolating them from the surrounding environment and inserting a control factor on the pharmacokinetics and pharmacodynamics thereof.

Preferably, the composition of the invention is used for topical use by application thereof to the skin of the face and/or body in need of treatment.

The composition can be for cosmetic or pharmaceutical use.

The cosmetic composition can be in solid, semi-solid or liquid form.

Suitable compositions in solid form include creams, gels, pomades, pastes, ointments.

Suitable compositions in liquid form include solutions, suspensions, lotions, serum, emulsions both oil in water and water in oil.

In a preferred embodiment, the formulation for local application is in the form of a liposome-based lotion.

The compositions of the invention can comprise excipients commonly used in the formulation of preparations for topical use such as preservatives, bactericidal agents, stabilizers, antioxidants, emulsifiers, buffers, humectants, dyes and other excipients commonly used in pharmaceutical or cosmetic preparation techniques, in quantities in line with those envisaged for common pharmaceutical or cosmetic formulations.

In the case of formulations in solution, suspension, lotion form, water is present as a diluent or solvent, optionally mixed with other liquids used for the formulation of cosmetic compositions such as for example alcohols, and glycols like propylene glycol.

Advantageously, the composition can contain a carrier of fat matter, such as for example liposomes or fatty acids used in the cosmetic formulations.

The composition of the invention can contain peptides in a quantity ranging from 0.0001% to 10%, by weight, based on the chemical-physical characteristics of the product, the type of activity desired, the type and extent of the area to be treated, the sex and the age of the user.

In accordance with certain embodiments, the cosmetic composition of the invention further comprises one or more active substances such as minerals, micronutrients, vitamins and optionally other biologically active substances.

By way of example, the composition may include one or more further components selected from among amino acids such as arginine, proline, glycine, lysine, polypeptides, hydroxy acids such as lactic, glycolic, salicylic, tartaric, malic acid etc., natural extracts or essential oils of plants, vitamins such as tocopherol, lipoic acid, retinol, panthenol, ascorbic acid, flavonoids etc., glycans such as hyaluronic acid, chondroitin sulfate, heparinoids etc., proteins such as wheat proteins, collagen, silicones, molecules of various kinds such as allantoin, caffeine, bisabolol, coenzyme Q10.

The composition of the invention finds application in the pharmaceutical and/or cosmetic field.

In accordance with another aspect, the present invention relates to the peptides of general formula (1) for use as a medicament.

According to one aspect the invention provides the peptides for use object of claim 8.

In accordance with some embodiments, the peptides of the invention and the compositions containing them find application in the treatment of subjects affected by Xeroderma Pigmentosum (XP) who have a genetic mutation that causes a deficit in DNA repair and are at high risk of developing malignant tumours. In the course of clinical trials, the composition described herein when applied topically has demonstrated to contribute to the elimination of CPDs at the application site and has demonstrated to be effective in preventing the development of actinic keratosis and carcinomas in the basal cells in XP patients.

The composition for topical use comprising one or more peptides, each other equal or different, of SEQ ID NO:1 or SEQ ID NO:2, SEQ ID NO:3-8, the salts thereof when applied to the skin crosses the stratum corneum and penetrates the deepest layers of the epidermis, accumulating in the keratinocytes and in the Langerhans cells. These properties are more marked when the peptides of the composition are carried by liposomes.

In accordance with certain aspects, the present invention provides a cosmetic composition comprising at least one peptide described herein and a cosmetically acceptable excipient or carrier for preventing or reducing the marks of skin aging in particular consequent to exposure to ultraviolet radiations.

In accordance with other aspects the present invention provides a cosmetic composition comprising at least one peptide described herein and a pharmaceutically acceptable excipient or carrier for the uses described herein.

In accordance with another aspect, the present invention provides a cosmetic kit for the treatment of the skin, in particular for depigmenting the skin as defined in claim 10.

In one embodiment, the depigmenting cosmetic kit comprises
- A composition for peeling skin, comprising an α-hydroxy acid such as for example glycolic acid, mandelic acid and kojic acid;
- A post-peeling buffer solution;
- The liposomal formulation *XP-1* (Ptd-1 (*WFKYYGKAIY*) 0.1% in liposomes, as per Example 1)
- A gel based on S-Arbutin and liposomes;
- A post-treatment sunscreen.

The liposomal composition XP-1 regenerates damaged skin cells by carrying out a repairing action

In addition, other important active functional ingredients of the kit are the glycolic acid, the mandelic acid and the kojic acid (inside the exfoliating solution), and S-Arbutin (inside the whitening formulation).

Glycolic acid is an α-hydroxy acid commonly used as a component of dermatological preparations with exfoliating action, thanks to its demonstrated capability of accelerating cell turnover of the stratum corneum.

Mandelic acid is also an α-hydroxy acid, with proven antibacterial as well as exfoliating properties, effective in improving skin texture, acne, premature skin aging and hyperpigmentation.

Kojic acid (AC) is a natural metabolite produced by fungi that has the capability of inhibiting the activity of tyrosinase in melanin synthesis.AC and the derivatives thereof are used in cosmetics as antioxidants, anti-proliferatives, antiinflammatories and skin lightening agents.

Also S-Arbutin, thanks to its action as an inhibitor of the enzyme tyrosinase, is an antimelanogenic substance, which therefore has a lightening and depigmenting action.

The following examples of embodiments of the present invention are given below by way of illustration.

### EXAMPLES

### Example 1

### Therapeutic application of the peptides in patients affected by Xeroderma Pigmentosus

The efficacy tests *in vitro* were conducted with 7 selected liposomal formulations, each containing a different peptide:
- **XP-1**:Ptd-1 (*WFKYYGKAIY*) 0.1% in liposomes;
- **XP-2:**Ptd-2 (Carboxy fluorescein- *WFKYYGKAIY*) 0.1% in liposomes;
- **XP-3**:Ptd-3 (*Palmitoyl-WFKYYGKAIY*) 0.1% in liposomes;
- **XP-5**:Ptd-5 (Palmitoyl- *WFKYYGK-Aib-IY)* 0.1% in liposomes;
- **XP-6**:Ptd-6 (Carboxy *fluorescein- WFKYYGK-Aib-IY)* 0.1% in liposomes;
- **XP-7**:Ptd-7 (*Aib-WFKYYGKAIY*) 0.1% in liposomes;
- **XP-8**:Ptd-1 (*WFKYYGKAIY*) 0.01% in liposomes.

The liposomal formulations were applied in efficacy tests on the epithelial cells KB-31 and primary fibroblasts isolated by biopsy belonging to patients affected by Xeroderma pigmentosum (XPC-F).

**The tests carried out comprise:**
a) Cell uptake test on epithelial-derived cell line KB-31 by flow cytometry. The sample XP-1 was compared to the liposomal formulation not containing peptides ( **XP-0** ), the liposomal formulation containing the enzyme endonuclease T4V ( **XP-T4V** ) and the liposomal formulation XP-2, containing the same peptide functionalized with carboxy fluorescein (CF).
b) Assessment of cellular uptake of the liposomes by XPC-F fibroblasts via live cell imaging using the liposomal formulation XP-2 containing the peptide functionalized with the fluorophore carboxy fluorescein (CF).
c) Assessment of the biological activity of liposomal formulations on:
   - XPC-F fibroblasts, by analysis of the transcriptional levels, with comparison between expanding cells, cells irradiated with UV radiation and not treated, irradiated cells treated with the liposomal formulation XP-T4V, and irradiated cells treated with the liposomal formulation XP-1;
   - on the epithelial cells KB-31, to test the biological activity of the liposomal formulations XP-1, XP-3, XP-5, XP-6, XP-7, XP-8, by assessing the levels of gene expression.
d) Assessment of the biological activity of liposomal formulations on XPC-F by cell viability test and cell cycle study, with comparison between non-irradiated cells, untreated irradiated cells, irradiated cells treated with the formulation XP-T4V, and cells irradiated and treated with the liposomal peptides.

### 1.1 The results of in vitro tests

### a) Uptake test on KB-31 keratinocytes.

The cellular uptake of the formulations XP-0, XP-1, XP-T4V and XP-2 was monitored by flow cytometry. For the analysis, the KB-31 cells were treated with the formulations at concentrations ranging from 10 to 50 µL/mL. After 72 hours from the start of the treatment, the cells were analysed using the BD FACSCanto II flow cytometer The analysis of the samples treated with formulation XP-2 showed a significant number of fluorescent cells (> 90%) confirming a marked cellular uptake of the peptide labelled with the fluorophore. It is also important to highlight that, after 72 hours from the treatment, the fluorescent signal is still significantly detectable.

### b) Assessment of the cellular uptake of liposomes by live cell imaging on XPC-F fibroblasts.

To verify the cellular uptake of the liposomes, the XPC-F cells were treated with a solution (20 µL/mL) containing the formulation XP-2 and immediately exposed to UV irradiation (300-800 nm), at a dose of 360 W/m² for 7 minutes using a solar simulator [SunTest CPS ⁺ (ATLAS, Urai, Italy)].The XPC-F cells treated with the same liposomal formulation and not irradiated were used as controls. 10 minutes after the end of irradiation, the cells were observed by confocal time-lapse microscopy, using a Cell-Observer (Zeiss) system equipped with an incubator chamber that guarantees controlled atmospheric conditions (37°C, 5% CO₂). Following the fluorescent signal, the XP-2 liposomes were monitored for 2 hours.

The XPC-F cells were found to have successfully absorbed the XP-2 liposomes and maintained the intracellular fluorescence during the 2-hour incubation time.

The study highlighted that the cells that were subjected to the treatment and not irradiated are able to internalize the liposomes; however, only after irradiation, due to the changes in the cellular environment caused by this stress, the liposomal structure is able to gradually release the peptide inside the nucleus and cytoplasm.

### c) Assessment of the biological activity of liposomal formulations on XPC-F fibroblasts and KB-31 keratinocytes by analysing transcriptional levels.

Subconfluent cell cultures of XPC-F were treated with the liposomal formulations XP-T4V and XP-1 (20µL/mL) and exposed to UV radiation (300-800 nm) at a dose of 360 W/m ² (SunTest CPS ⁺ - ATLAS, Urai, Italy) for 7 minutes.

In parallel, subconfluent cultures of KB-31 epithelial cells were treated with the liposomal formulations XP-1, XP-3, XP-5, XP-6, XP-7, XP-8 (20µL/mL) and irradiated with UV rays (300-800 nm, dose of 360 W/m ², 7 minutes) using the SunTest CPS simulator ⁺- ATLAS, Urai, Italy.

At the end of the irradiation, the XPC-F and KB-31 cells were kept in culture for 6 hours in the solution containing the liposomes. Cells not treated with any formulation and not irradiated as well as cells subjected to irradiation in the absence of treatment with liposomal formulations were used as control of each cell type.

The total cellular RNA was extracted at the end of 6 hours using the TRI Reagent solution (Zymo Research, USA), following the protocol indicated by the manufacturer. Following quantification with NanoDrop 2000 (Thermo-Fisher Scientific, Inc., Waltham, MA), the samples were analysed by one-step qRT-PCR using the qPCRBlO SYGREEN kit (Resnova, Italy), oligonucleotides (Invitrogen^{™} Life Technologies ) and the Mic Real-time PCR (Bio Molecular Systems) system.

This study served to assess the transcriptional levels of several genes involved in signalling pathways involved in irradiation stress. The genes analysed are as follows:
- *BCL2* (B-cell lymphoma/leukemia-2): is an anti-apoptotic gene that prevents or reduces cellular apoptosis activated by a wide range of stimuli, including UV radiation.
- *CCNB1* (cyclin B1): it is a regulatory subunit of cyclin-dependent kinase (CDK) necessary for the correct control mechanism of the cell cycle progression from phase G2 to phase M. The study thereof was taken into consideration because in the literature it is known that UV radiations cause the cell cycle to be blocked in the phase G2.
- *p53:* it is a gene of the tumour suppressor family, necessary for stimulating an efficient nucleotide excision repair (NER) following UV rays induced DNA damage and implicated in the induction of apoptosis of cells unable to repair the damage induced by UV rays.
- *p21:*it is a cell cycle regulator which is activated at the nuclear level by p53.This protein induces a slowing down of the cell cycle. A down-regulation of this gene leads to a greater advancement in the cell cycle.
- *p27:* it is an indicator of genomic stability which is reduced in case of irradiation.
- *p66Shc:* it is an indicator of oxidative stress which is active at the mitochondrial level; when activated, the *p66Shc* protects cell from reactive oxygen species (ROS).
- *ErbB3 (HER3):* coding for EGF receptor 3. The loss of these receptors leads to the onset of negative effects on the skin and the regenerative processes thereof.
- *TNFR1:* it is a gene coding for the Tumor Necrosis Factor-α (TNF-α) receptor 1, involved in supporting the inflammatory response through apoptosis.
- *TNFR2:* gene coding for the tumor necrosis factor-α (TNF-α) receptor 2, involved in the inflammatory response as a positive regulator of cell survival.
   - *FGFR2:* it is the gene coding for the Fibroblasts Growth Factor 7 (FGF7) receptor 2. It acts as an action mediator of FGF7 and appears to be upregulated in numerous tumors. Its down-regulation has suppressive effects on tumor growth.

The results reported in the following tables compare the expression levels of the markers in the cells treated with the selected liposomal formulations, with respect to the cells not treated and subjected to equal UV irradiation.

| **XPC-F** | **XP-T4V** | **XP-1** |
|---|---|---|
| ***BCL2*** | NE | NE |
| ***CCNB1*** | NE | Down-R |
| ***p53*** | Up-R | NE |
| ***p21*** | Down-R | Down-R |
| ***p27*** | Down-R | Down-R |
| ***p66Shc*** | Up-R | NE |
| ***ErbB3 (HER3)*** | Up-RNE | NE |
| ***TNFR1*** | NE | Down-R |
| ***TNFR2*** | NE | Up-R |
| ***FGFR2*** | NE | Down-R |

| | | |
|---|---|---|
| *Up-regulation:Up-R; Down-regulation:Down-R; No effect:NE.* | | |

| **KB-31** | **XP-1** | **XP-3** | **XP-5** | **XP-6** | **XP-7** | **XP-8** |
|---|---|---|---|---|---|---|
| ***BCL2*** | Up-R | Up-R | Up-R | Up-R | Up-R | NE |
| ***CCNB1*** | NE | Up-R | Up-R | NE | NE | NE |
| ***p53*** | NE | NE | NE | NE | NE | NE |
| ***p21*** | Down-R | Down-R | Down-R | Down-R | Down-R | Down-R |
| ***p27*** | Up-R | NE | NE | NE | NE | NE |
| ***p66Shc*** | NE | NE | NE | NE | NE | NE |
| ***ErbB3 (HER3)*** | Down-R | Down-R | Down-R | Down-R | Down-R | Down-R |
| ***TNFR1*** | NE | NE | Up-R | NE | NE | NE |
| ***TNFR2*** | NE | NE | NE | NE | NE | NE |
| ***FGFR2*** | *NO-Exp* | *NO-Exp* | *NO-Exp* | *NO-Exp* | *NO-Exp* | *NO-Exp* |

| | | | | | | |
|---|---|---|---|---|---|---|
| *Up-regulation:Up-R; Down-regulation:Down-R; No effect:NE; NO-Exp:No Expression.* | | | | | | |

In conclusion, the collected data demonstrated that UV irradiation triggers a panel of cellular mechanisms that control apoptosis, cell cycle, regulation of oxidative stress levels and responsiveness to growth factors. The treatment of cells with liposomal peptides has a well-defined impact on the gene expression levels for some of the genes analysed.

The formulations XP-T4V and XP-1 have effects that are different in the two cell types, underlining how fibroblasts and epithelial cells synergistically play different roles in skin homeostasis.

In particular, the analysis conducted on XPC-F has highlighted how the down-regulation of factors that negatively regulate the progression of the cell cycle, as well as the proliferation (such as p21 and p27) is counterbalanced by the up-regulation of tumour suppressors, having a protective effect against the onset of cancer, emphasizing the safety of the formulations used.

The study carried out on KB-31 highlights similar effects that were obtained from the liposomal formulations, which are more marked in the formulations XP-3 and XP-5.

### d) Assessment of the biological activity of liposomal formulations on XPC-F by cell viability test.

The subconfluent cultures of XPC-F were subjected to treatment with the liposomal formulations XP-T4V, XP-1, XP-3, XP-5, XP-6, XP-7 and XP-8 (20 µL/mL) and subjected to UV irradiation (300-800 nm) at a dose of 360 W/m² for 7 minutes using the SunTest CPS simulator ⁺ (ATLAS, Urai, Italy).

The fibroblasts maintained in culture with the same timing without treatment/irradiation and the cells not treated with liposomal formulations but irradiated were used as controls.

At the end of the irradiation, the cells were maintained in culture in complete growth medium for 24 hours, 48 hours and 72 hours, before proceeding to the analysis of cell viability.

The fibroblasts, detached from the plate with trypsin-EDTA, were treated using the BD Cell Viability kit (BD, New Jersey, USA): a 5-minute incubation at room temperature was carried out with a solution of Thiazole Orange (TO) (42µ mol/L) and a solution of Propidium Iodide (PI) (4.3 mmol/L).At the end of this procedure, the samples were analysed by BD FACSCanto II flow cytometer.

The results obtained are shown below:

| **24h** | **-LIPO/-UV** | **-LIPO/+UV** | **XP-T4V** | **XP-1** | **XP-3** | **XP-5** | **XP-6** | **XP-7** | **XP-8** |
|---|---|---|---|---|---|---|---|---|---|
| **Live cells** | 80.0% | 73.8% | 73.6% | 63.1% | 59.4% | 58.3% | 46.6% | 49.9% | 73.6% |
| **Apoptotic cells** | 18.1% | 26.3% | 23.2% | 33.8% | 38.9% | 41.3% | 50.2% | 48.3% | 26.2% |

| **48h** | **-LIPO/-UV** | **-LIPO/+UV** | **XP-T4V** | **XP-1** | **XP-3** | **XP-5** | **XP-6** | **XP-7** | **XP-8** |
|---|---|---|---|---|---|---|---|---|---|
| **Live cells** | 86.7% | 76.7% | 71.0% | 76.3% | 72.2% | 69.0% | 57.9% | 70.2% | 67.5% |
| **Apoptotic cells** | 10.9% | 14.1% | 15.1% | 10.6% | 15.5% | 16.9% | 20.7% | 15.7% | 17.6% |

| **72h** | **-LIPO/-UV** | **-LIPO/+UV** | **XP-T4V** | **XP-1** | **XP-3** | **XP-5** | **XP-6** | **XP-7** | **XP-8** |
|---|---|---|---|---|---|---|---|---|---|
| **Live cells** | 62.5% | 48.0% | 47.3% | 41.1 % | 39.9% | 42.4% | 46.4% | 57.1 % | 45.6 % |
| **Apoptotic cells** | 33.9% | 47.7% | 47.8% | 41.5% | 56.2% | 52.3% | 47.3% | 47.3% | 44.7% |

| | | | | | | | | | |
|---|---|---|---|---|---|---|---|---|---|
| *-LIPO*/*-UV:No liposomal formulations*/*No irradiation; -LIPO*/*+UV:No liposomal formulations*/*Yes irradiation.* | | | | | | | | | |

In this study, the cells showed a reduced cell viability 24 hours after UV irradiation, regardless of the pre-treatment with liposomal formulations, however the lower percentage of apoptotic cells was obtained using the formulations XP-T4V, XP-1 and XP-8.

After 48 h from irradiation, the number of cells in apoptosis was found to be lower in the cultures treated with the liposomal formulations, especially with XP-1, compared to those in the irradiated sample in the absence of liposomal formulations. Finally, 72 hours after irradiation, the fibroblasts that underwent pretreatment with the tested formulations show results comparable to those obtained in the irradiated cells in the absence of formulations.

The analysis of the cell cycle was performed using subconfluent cultures of XPC-F, treated with the liposomal formulations XP-T4V and XP-1 (20 µL/mL).Subsequently, the cells were irradiated as reported above. Untreated fibroblasts with liposomal/non-irradiated formulations, and cells not treated with liposomal formulations/subjected to irradiation were used as controls.

Following irradiation, the cells were cultured in growth medium for 24 hours, 48 hours and 72 hours. At each time, sample assessment was performed by BD FACSCanto II flow cytometer.

The study of the cell cycle of irradiated fibroblasts, 24 hours after irradiation, highlighted that both formulations (XP-T4V and XP-1) are able to slow down the cells in their cell cycle, probably in order to favour the development of DNA repair processes. In particular, the formulation XP-1 induced an increase in the percentage of cells in G2 stage. 48 hours after irradiation, a higher percentage of cells in S and G2 phase emerged in the samples treated with both liposomal formulations, suggesting a cell cycle being unblocked. The same effects shown at 48 hours also emerged 72 hours after irradiation. It can therefore be hypothesized that the peptide function at the cellular level is specifically correlated with a prolongation of the phase preceding apoptosis, allowing the cell a longer interval of time for damage repair, with a final effect of prevention of apoptosis.

### Example 2

### 1 Clinical study design

The cosmetic kit described in example 4 was subjected to a clinical study in order to assess the efficacy and tolerability thereof on the basis of optical colorimetry tests, videodermatoscopy and self-assessment questionnaire on the subjects involved. The clinical study was conducted in open label, on a single centre, under dermatological supervision in the course of 4 weeks. The intra-subject comparison was envisaged ( *half-face* method) between the efficacy of the peeling procedure ( *Peeling solution* + *Post-peeling Buffer solution* + *SPF 50+ sunscreen*) with or without the addition of both depigmenting products *XP-1* and *Liposomes S Arbutine Gel.*

The study was conducted on a total of 20 female subjects, aged 39-60 years (mean = 50) with hyperpigmented skin spots on the face.

The superficial chemical peeling was performed bilaterally on the whole face (excluding the eyelids) of the subjects using the *Peeling solution* and an appropriate brush. After the appearance of an erythema and a slight whitening effect on the treated skin, the reaction was neutralized using the *Post-peeling Buffer solution.*

The treatment was continued by the subjects independently at home, who applied the following products to one side of the face, twice a day (in the morning and in the evening, preferably at the same time):
- the liposomal formulation *XP-1* (directly on the hyperpigmentation points selected by the dermatologist during the basic check-up),
- the *Liposomes S Arbutine Gel* (on the entire surface of the same half-face). Finally, the post-treatment lightening sunscreen ( *SPF 50+*) was applied bilaterally in the morning and evening to the entire face for the whole duration of the study. For determining the depigmenting activity of the studied treatment, the measurement of the brightness of the skin as well as the colour and size (area and perimeter) of the selected hyperpigmented points was performed bilaterally, by means of optical colorimetry and image analysis at the following times:
   □ baseline (initial check-up - T0, before the peeling procedure)
   □ 2 weeks after peeling (intermediate check-up - T2)
   □ 4 weeks after peeling (intermediate check-up - T4).

Two dropouts occurred during the study; volunteers Nos. 10 and 15 prematurely interrupted the process, for personal reasons. The statistical analysis was performed on 18 subjects, who completed the study as directed by the protocol. No other major events that might have interfered with the test results occurred during the study period.

The activity of the depigmentation kit in each time interval was expressed in absolute values with respect to baseline (T0) and with respect to the side of the control face subjected to the peeling procedure only.

Data processing was performed as follows:

### • Assessment with respect to baseline conditions (T0)

Non-parametric test (Friedman test), in cases where the hypothesis of normality was rejected by the Shapiro-Wilk test of normality (threshold at 5%); or parametric test (Anova test for repeated measurements), in cases where the hypothesis of normality was confirmed; both tests were followed in case of a statistically significant result of the Holm-Sidak Adjusted tests.

### • Comparison between the two treatments (peeling + depigmenting treatment vs peeling) at T0, T2 and T4

Non-parametric test (Wilcoxon test), in cases where the hypothesis of normality was rejected by the Shapiro-Wilk test of normality (threshold at 5%); or parametric test (Paired t test), in cases where the hypothesis of normality was confirmed.

### 2 Results of the clinical study: efficacy of the product

### 2. 1 Assessment of skin colour (optical colorimetry)

The following tables summarize the percentages of variation of the parameters L*, a* and b* which represent respectively skin brightness (L*), redness (a*) and pigmentation (b*), measured on non-hyperpigmented skin (normal skin ) and on hyperpigmented skin (directly on the spots).(Figure X and X)

| **Results with:Peeling + Depigmenting treatment** | | | | | | | | | | | | |
|---|---|---|---|---|---|---|---|---|---|---|---|---|
| *Normal skin* | | | | | | | *Hyperpigmented skin (with spots)* | | | | | |
| % variation vs baseline | | | | | | | % variation vs baseline | | | | | |
| **L*** | | **a*** | | **b*** | | | **L*** | | **a*** | | **b*** | |
| **T2** | **T4** | **T2** | **T4** | **T2** | **T4** | | **T2** | **T4** | **T2** | **T4** | **T2** | **T4** |
| 1.4% (*)( ^{#} ) | 1.5 % | -3.4% | -5.7% | -5.8% | -5.1% (*) | | -0.1% | 0.4% | 1.0% | -4.3% | -8.6% | -8.7% (*) |
| **Results with:Peeling** | | | | | | | | | | | | |
| *Normal skin* | | | | | | | *Hyperpigmented skin (with spots)* | | | | | |
| % variation vs baseline | | | | | | | % variation vs baseline | | | | | |
| **L*** | | **a*** | | **b*** | | | **L*** | | **a*** | | **b*** | |
| **T2** | **T4** | **T2** | **T4** | **T2** | **T4** | | **T2** | **T4** | **T2** | **T4** | **T2** | **T4** |
| -0.4% | 1.2 % | -0.4% | -0.1% | -3.5% | -0.8% | | -0.1% | -0.2% | 5.8% | -1.4% | -6.9% | -7.6% (*) |

| | | | | | | | | | | | | |
|---|---|---|---|---|---|---|---|---|---|---|---|---|
| (*) p<0.05 Holm-Sidak Adjusted test vs T0.(^{#}) p<0.01 Wilcoxon signed rank test vs T0. | | | | | | | | | | | | |

On non-hyperpigmented skin (normal skin) subjected to peeling + depigmenting treatment it was highlighted starting from the control at T2 as follows:
- a clinically/statistically significant improvement in skin brightness (L*), statistically greater than the result obtained on the side of the face subjected to peeling only;
- a reduction in skin redness (a*), not detectable with peeling alone,
- an important reduction in skin pigmentation (b*) clinically more marked than the one highlighted by peeling alone.

These results show that the comprehensive treatment accelerated and improved the performance of the lightening peeling.

At the end of the entire study, the complexion of the skin on both sides of the face generally appeared brighter and more homogeneous.

Regarding hyperpigmented skin (with spots) , although no statistically significant difference was found between the two sides of the face, the depigmenting activity was more evident on the side treated with *XP-1* and *Liposomes S Arbutine Gel,* compared to the side treated with peeling only, with a marked reduction in the colouring and visibility of skin spots/pigmentation (b*).

### 2.2 Image analysis of skin spots (videodermatoscopy)

The following tables summarize the percentage values of reduction in the area and perimeter of the spots, measured by image analysis:

| **Results with:Peeling + Depigmenting treatment** | | | | | **Results with:Peeling** | | | |
|---|---|---|---|---|---|---|---|---|
| % variation vs baseline | | | | | % variation vs baseline | | | |
| *Area* | | *Perimeter* | | | *Area* | | *Perimeter* | |
| **T2** | **T4** | **T2** | **T4** | | **T2** | **T4** | **T2** | **T4** |
| -10.4% (*) | -14.7% (*) | -8.6% (*) | -7.2% (*) | | -12.3% (*) | -12.2% (*) | -4.2% | -5.6% |

| | | | | | | | | |
|---|---|---|---|---|---|---|---|---|
| (*) p<0.05 Holm-Sidak Adjusted test vs T0. | | | | | | | | |

The results obtained highlighted for the comprehensive depigmenting treatment *(Peeling solution +formulation XP-1* and *Liposomes S Arbutine Gel*)*,* starting from control T2:
- a statistically significant reduction in the area of dark spots compared to T0, comparable to the one obtained for peeling alone.
   - a more marked and statistically significant reduction in the perimeter of dark spots compared to T0, indicating a more uniform reduction in hyperpigmentation of the spots.

### 2.3 Self-assessment questionnaire posed to the subjects involved

### EFFICACY ASSESSMENT

For 66% of the subjects the spots were less evident (average 33%, marked 22%, very marked 11% effect) and for 55.5% of reduced size (average 38.8%, marked 16.7% effect) on the side subjected to the comprehensive exfoliating and lightening treatment, with a more marked global depigmenting effect than the side of the face subjected to peeling only for 44% of cases. The improvement in skin brightness and skin dryness was found to be comparable between the 2 treatments.

### COSMETIC ACCEPTABILITY ASSESSMENT

The cosmetic acceptability of the products (*XP-1 Liposomes S Arbutine Gel,* post-treatment lightening sunscreen) was generally good. In particular, 100% of the volunteers judged good-excellent:
- *Liposomes S Arbutine Gel* - in terms of: colour (94% of the subjects), perfume before and after application (72% of the cases), texture (78% of the subjects), spreadability (94% of the subjects), absorption (61% of the volunteers), effect on the skin (72% of the subjects) and absence of residues after application (83% of the volunteers).
- *Formulation XP-1* - in terms of: colour (94.5% of the subjects), perfume before and after application (77.8% and 72.2% of the cases respectively), texture (89% of the subjects), spreadability (83% of the subjects), absorption (78% of the volunteers), effect on the skin (83% of the subjects) and absence of residues after application (94.5% of the volunteers).
- *Post-treatment lightening sunscreen* - in terms of: colour (94% of the subjects), perfume before and after application (77.8% of the subjects), texture (100% of the subjects), spreadability (83% of the subjects), absorption (72% of the volunteers), effect on the skin (77.8% of the subjects) and absence of residues after application (83% of the volunteers).

### 3 Tolerability assessment

The judgment of the investigator and of the volunteers on the tolerability of the tested products was good/excellent in 100% of the cases treated; no study treatment-related adverse events occurred during study 3.

### 4 Study conclusion

The results of this study confirmed the superior efficacy of the comprehensive depigmenting kit, comprising the products *XP-1* and *Liposomes S Arbutine Gel,* if compared with the peeling treatment only, highlighting higher lightening and "anti-spot" performance.

No adverse events or reactions occurred during the study; the experimenter judged the tolerability of the tested treatment to be good/excellent.

### Example 3

Formulations containing liposomes of Example 1.

The formulations reported in example 1 were prepared starting from a basic composition of phospholipids in water, stabilized using preservatives, containing the peptides SEQ ID NO:1-8 embedded inside the liposomal system.

The lipid concentration of the formulation is 100 mM, the encapsulated peptide concentration is 1 mM.

The INCI of the formulation is as follows: Aqua, Lecithin, Polisorbate 20, Ethylhexylglycerin, 1,2-Hexanediol, Caprylyl glycol, Alcohol denat, *Peptide SEQ ID NO:1-7,* Tocopherol, Tropolone.

### Example 4

Depigmenting cosmetic kit comprising 5 products (components) with specific activity.

The *Peeling Solution* is the preparation intended for skin exfoliation, it exerts its action thanks to its content of two α-hydroxy-acids combined with kojic acid. The INCI of the formulation is as follows: Aqua, Glycolic Acid, Mandelic Acid, Kojic Acid, Polysorbate 20, Ethylhexyl Glycerin, 1,2-Hexanediol, Caprylyl Glycol, Tropolone. The *Post-peeling Buffer Solution* consists of a buffer and is aimed at neutralizing the peeling solution after the treatment is completed. The INCI thereof is: Aqua, Pentylene Glycol, Disodium Phosphate, Sodium Phosphate.

The depigmenting cosmetic kit also contains a high protection sun product, the *SPF 50+ sunscreen.* The efficiency thereof in protecting the skin from UVA and UVB rays is achieved using 3 different sunscreens in combination. The INCI thereof is as follows:
Aqua, Caprylic/Capric Triglyceride, Pentylene Glycol, Ethylhexyl Stearate, Ethylhexyl Methoxycinnamate, Titanium Dioxide, Methylene Bis-Benzotriazolyl Tetramethylbutylphenol, Propylene Glycol, Arachidyl Alcohol, Isononyl Isononanoate, Butyl Methoxydibenzoylmethane, Behenyl Alcohol, Sodium Hyaluronate, Arachidyl Glucoside, Polyacrylamide, Urea, Silica, C13-14 Isoparaffin, Dimethicone, Sodium Chloride, Laureth-7.

Finally, the kit contains two products with a targeted depigmenting action. The first one, called *Depigmenting formulation XP-1,* reported in example 3, helps damaged cells in the step of repair of the damage caused by irradiation.

The second product is the *Liposomes S Arbutine Gel,* the lightening action thereof on the skin is obtained thanks to arbutin, a known inhibitor of melanin.

The INCI of the product is: Aqua, Lecithin, Arbutin, Glycerin, Polisorbate 20, Ethylhexylglycerin, 1,2-Hexanediol, Caprylyl glycol, Alcohol denat, Tocopherol, Tropolone.

### Example 5

### Formulation in lotion form for cosmetic use

The liposomal formulation containing the peptide with formula SEQ ID NO:1 has been included in an after-sun soothing lotion, which performs a triple soothing, regenerating and anti-aging action on the skin. The INCI of this product comprises: aqua, glycerin, cetearyl alcohol, *persea gratissima* oil, *prunus amygdales* oil, ceteareth-25, polisorbate 20, menthol, *calendula officinalis* extract, sodium hyaluronate, urea, peptide SEQ ID NO:1.

## Claims

1. A peptide or salt thereof, consisting of the amino acid sequence represented by the SEQ ID NO:1:
R. ¹ -Y-Xaa ¹ -Xaa ² - Xaa ³ -Xaa ⁴ - Xaa ⁵ -Xaa ⁶ - Xaa ⁷ -Xaa ⁸ - Xaa ⁹ -Xaa ¹⁰
wherein :
Xaa1 is Trp, Phe or Tyr;
Xaa2 is Trp, Phe or Tyr;
Xaa3 is Lys;
Xaa4 is Tyr, Trp or Phe;
Xaa5 is Tyr, Trp or Phe;
Xaa6 is Gly or Ala;
Xaa7 is Lys;
Xaa8 is Ala or Aib;
Xaa9 is Ile, Leu or Ala;
Xaa10:Tyr;
Y, if present, is Aib
and wherein
**R¹**, if present, is H, CF (carboxy fluorescein), palmitoyl (CH₃(CH₂)₁₄CO-), oleyl (CH₃(CH₂)₇(CH)₂(CH₂)₇ CO-), stearyl (CH₃(CH₂)₁₆CO-), linoleyl (CH₃(CH₂)₄(CH)₂CH₂(CH)₂(CH₂)₇CO-), or linolenyl (CH₃CH₂(CH)₂CH₂(CH)₂CH₂(CH)₂(CH₂)₇CO-).

2. The peptide according to claim 1 selected from the group consisting of
| SEQ ID NO: | | Sequence |
|---|---|---|
| 3 | | WFKYYGKAIY |
| 4 | | CF-WFKYYGKAIY |
| 5 | | Pal-WFKYYGKAIY |
| 6 | | WFKYYGK-Aib-IY |
| 7 | | Pal-WFKYYGK-Aib-IY |
| 8 | | CF-WFKYYGK-Aib-IY |
| 2 | | Aib-WFKYYGKAIY |
or mixtures thereof.

3. The peptide according to claim 1 or 2, consisting of the amino acid sequence from N-terminus end to C-terminus end represented by the SEQ ID NO:3.

4. A composition comprising one or more peptides, each other equal or different, according to any one of claims 1 to 3, and a physiologically acceptable carrier.

5. The composition according to claim 4 wherein said carrier comprises liposomes as a carrier for topical application.

6. The composition according to claim 4 or 5 in lotion form for topical application wherein peptides are embedded and/or carried by liposomes.

7. A peptide according to any one of claims 1-3 or a composition according to any one of claims 4-6 for use as a medicament.

8. The peptide according to any one of claims 1-3 or the composition according to any one of claims 4-6 for use in the treatment of a disease caused by DNA damaged by UV-ray exposure, in particular for the treatment of xeroderma pigmentosum or a cancerous or pre-cancerous skin condition.

9. A cosmetic use of a peptide according to any one of claims 1-3 or of a composition according to any one of claims 4-6 for the treatment of a cosmetic imperfection of skin, in particular skin spots or dyschromia or facial wrinkles.

10. A kit for the cosmetic treatment of the skin comprising a depigmenting cosmetic kit comprising
- a cosmetic composition for peeling skin, preferably comprising a α-hydroxy acid preferably glycolic acid, mandelic acid or kojic acid;
- a composition according to any one of claims 4-6;
- a composition preferably in gel form comprising liposomes and S-arbutin and liposomes; and optionally
- a post-peeling buffer composition preferably in solution form;
- a composition for UV-ray protection preferably in cream form.

## Patentansprüche

1. Ein Peptid oder dessen Salz, welches aus der Aminosäuresequenz besteht, die durch SEQ ID NO:1 dargestellt wird:
R. ¹-Y-Xaa¹-Xaa² - Xaa³ - Xaa⁴ - Xaa⁵ - Xaa⁶ - Xaa⁷- Xaa⁸ -Xaa⁹-Xaa¹⁰
wobei:
Xaa1 Trp, Phe oder Tyr ist
Xaa2 Trp, Phe oder Tyr ist;
Xaa3 Lys ist;
Xaa4 Tyr, Trp oder Phe ist;
Xaa5 Tyr, Trp oder Phe ist;
Xaa6 Gly oder Ala ist;
Xaa7 Lys ist;
Xaa8 Ala oder Aib ist;
Xaa9 Ile, Leu oder Ala ist;
Xaa10 : Tyr;
Y, falls vorhanden, Aib ist
und wobei
**R¹**, falls vorhanden, H, CF (Carboxyfluorescein), Palmitoyl (CH₃(CH₂)₁₄CO-), Oleyl (CH₃(CH₂)₇(CH)₂(CH₂)₇CO-), Stearyl (CH₃(CH₂)₁₆CO-), Linoleyl (CH₃(CH₂)₄(CH)₂CH₂(CH)₂(CH₂)₇(CO-) oder Linolenyl (CH₃CH₂(CH)₂CH₂(CH₂)CH₂(CH)₂(CH₂)₇(CO-) ist.

2. Das Peptid gemäß Anspruch 1, ausgewählt aus der Gruppe , welche aus Folgendem besteht:
| SEQ ID NO: | Sequenz |
|---|---|
| 3 | WFKYYGKAIY |
| 4 | CF-WFKYYGKAIY |
| 5 | Pal-WFKYYGKAIY |
| 6 | WFKYYG-Aib-IY |
| 7 | Pal-WFKYYGK-Aib-IY |
| 8 | CF-WFKYYGK-Aib-IY |
| 2 | Aib-WFKYYGKAIY |
oder deren Mischungen.

3. Das Peptid gemäß Anspruch 1 oder 2, welches aus der Aminosäuresequenz vom N-Terminus Ende bis C-Terminus Ende besteht und durch die Sequenz SEQ ID NO:3 dargestellt wird.

4. Eine Rezeptur, die ein oder mehrere Peptide umfasst, welche jeweils gleich oder unterschiedlich sind, gemäß einem beliebigen der Ansprüche von 1 bis 3, sowie einen physiologisch akzeptablen Träger.

5. Die Rezeptur gemäß Anspruch 4, wobei der genannte Träger jeweils Liposome als Träger zur topischen Anwendung umfasst.

6. Die Rezeptur gemäß Anspruch 4 oder 5 in Form einer Lotion zur topischen Anwendung , wobei die Peptide jeweils eingeschlossen sind und/oder durch entsprechende Liposome getragen werden.

7. Ein Peptid gemäß einem beliebigen der Ansprüche 1-3 oder eine Rezeptur gemäß einem beliebigen der Ansprüche 4-6 bei seiner Anwendung als Medikament.

8. Das Peptid gemäß einem beliebigen der Ansprüche 1-3 oder die Rezeptur gemäß einem beliebigen der Ansprüche 4-6 bei Anwendung im Rahmen der Behandlung einer Krankheit, welche durch durch UV-Strahlenbelastung geschädigtes DNA verursacht wurde, und zwar insbesondere zur Behandlung von Xeroderma pigmentosum bzw. eines kanzerösen oder präkanzerösen Hautzustands.

9. Eine kosmetische Anwendung eines Peptids gemäß einem beliebigen der Ansprüche 1-3 oder einer Rezeptur gemäß einem beliebigen der Ansprüche 4-6 für die Behandlung einer kosmetischen Unvollkommenheit des Hautbilds, insbesondere von Flecken oder Dyschromien oder Faltenbildung im Gesicht.

10. Ein Kit für die kosmetische Behandlung der Haut einschließlich eines kosmetischen Depigmentierungs-Kits, welches jeweils Folgendes umfasst:
- Eine kosmetische Rezeptur für das Hautpeeling, die vorzugsweise eine α-hydroxy-Säure, vorzugsweise Glykolsäure, Mandelsäure oder Kojisäure, umfasst;
- Eine Rezeptur gemäß einem beliebigen der Ansprüche 4-6;
- Eine Rezeptur, vorzugsweise in Form eines Gels, welche jeweils Liposome und S-Arbutin und Liposome umfasst; sowie wahlweise
- Eine Post-Peeling-Pufferrezeptur, vorzugsweise in Form einer Lotion;
- Eine UV-Strahlenschutz-Rezeptur, vorzugsweise in Form einer Creme.

## Revendications

1. Peptide ou un sel de celui-ci, consistant en la séquence d'acides aminés représentée par la SEQ ID NO: 1 :
R. ¹-Y-Xaa ¹-Xaa ²- Xaa ³-Xaa ⁴- Xaa ⁵-Xaa ⁶- Xaa ⁷-Xaa ⁸- Xaa ⁹-Xaa ¹⁰ dans lequel :
Xaa1 est Trp, Phe ou Tyr ;
Xaa2 est Trp, Phe ou Tyr ;
Xaa3 est Lys ;
Xaa4 est Tyr, Trp ou Phe ;
Xaa5 est Tyr, Trp ou Phe ;
Xaa6 est Gly ou Ala ;
Xaa7 est Lys ;
Xaa8 est Ala ou Aib ;
Xaa9 est Ile, Leu ou Ala ;
Xaa10:Tyr ;
Y, le cas échéant, est Aib
et dans lequel
**R¹**, le cas échéant, est H, CF (carboxy fluorescéine), palmitoyl (CH₃(CH₂)₁₄CO-), oléyle (CH₃(CH₂)₇(CH)₂(CH₂)₇CO-), stéaryle (CH₃(CH₂)₁₆CO-), linoléyle (CH₃(CH₂)₄(CH)₂CH₂(CH)₂(CH₂)₇C0-), ou linolényle (CH₃CH₂(CH)₂CH₂(CH)₂CH₂(CH)₂(CH₂)₇CO-).

2. Peptide selon la revendication 1, sélectionné dans le groupe consistant en
| SEQ ID NO: | Séquence |
|---|---|
| 3 | WFKYYGKAIY |
| 4 | CF-WFKYYGKAIY |
| 5 | Pal-WFKYYGKAIY |
| 6 | WFKYYG-Aib-IY |
| 7 | Pal-WFKYYGK-Aib-IY |
| 8 | CF-WFKYYGK-Aib-IY |
| 2 | Aib-WFKYYGKAIY |
ou leurs mélanges.

3. Peptide selon la revendication 1 ou 2, consistant en la séquence d'acides aminés de l'extrémité N-terminale à l'extrémité C-terminale représentée par la SEQ ID NO:3.

4. Composition comprenant un ou plusieurs peptides, tous égaux ou différents, selon l'une quelconque des revendications 1 à 3, et un support physiologiquement acceptable.

5. Composition selon la revendication 4, dans laquelle ledit support comprend des liposomes en tant que support pour une application topique.

6. Composition selon la revendication 4 ou 5 sous forme de lotion pour application topique, dans laquelle les peptides sont incorporés et/ou transportés par des liposomes.

7. Peptide selon l'une quelconque des revendications 1 à 3 ou une composition selon l'une quelconque des revendications 4 à 6 pour une utilisation en tant que médicament.

8. Peptide selon l'une quelconque des revendications 1 à 3 ou la composition selon l'une quelconque des revendications 4 à 6 pour une utilisation comme traitement d'une maladie causée par l'ADN endommagé par l'exposition aux rayons UV, en particulier pour le traitement du xeroderma pigmentosum ou d'une affection cutanée cancéreuse ou précancéreuse.

9. Utilisation cosmétique d'un peptide selon l'une quelconque des revendications 1 à 3 ou d'une composition selon l'une quelconque des revendications 4 à 6 pour le traitement d'une imperfection cosmétique de la peau, en particulier les taches cutanées ou la dyschromie ou les rides du visage.

10. Kit pour le traitement cosmétique de la peau comprenant un kit cosmétique dépigmentant comprenant
- une composition cosmétique pour le peeling de la peau, comprenant de préférence un acide α-hydroxy, de préférence un acide glycolique, un acide mandélique ou un acide kojique ;
- une composition selon l'une quelconque des revendications 4 à 6 ;
- une composition de préférence sous forme de gel comprenant des liposomes et de la S-arbutine et des liposomes ; et éventuellement
- une composition tampon post-peeling, de préférence sous forme de solution ;
- une composition pour la protection contre les rayons UV, de préférence sous forme de crème.
